# EUROPEAN PATENT APPLICATION

(11) **EP 4 212 200 A1**
(43) Date of publication of application: **19.07.2023**
(21) Application number: 21875471.1
(22) Date of filing: 27.09.2021
(51) Int. Cl.: A61M 29/00, A61B 18/14

(54) **MEDICAL DEVICE SYSTEM AND ELECTRODE CONTACT DETECTION METHOD**

(30) Priority: 30.09.2020 JP 2020164556
(71) Applicant: TERUMO Kabushiki Kaisha, Tokyo 151-0072 (JP)
(72) Inventor: TAKAHASHI, Yusuke, Ashigarakami-gun, Kanagawa 259-0151 (JP)
(74) Representative: Casalonga
(86) International application number: PCT/JP2021/035234
(87) International publication number: WO 2022/071169

(57) **Abstract**

Provided are an electrode contact detection method and a medical device system that, when there are three or more electrodes, are capable of reliably detecting the state of contact of each electrode with respect to bio tissue. Said system comprising a medical device (10) having three or more electrodes (22) that are inserted in a living organism and a measurement unit (60) that measures impedance between the electrodes (22), wherein the measurement unit (60) is configured to select any one of the electrodes (22) and perform a measurement operation for measuring the impedance between one end of the selected electrode (22) and one parallel circuit end of all of the electrodes (22) aside from the selected electrode (22) by selecting all of the electrodes (22) one by one..

## Description

### Technical Field

The present invention relates to a medical device system including a medical device that applies energy to a biological tissue and an electrode portion contact detection method.

### Background Art

As a medical device, there is known one in which an electrode portion is disposed on an expansion body that expands and contracts in a biological body, and treatment is performed by ablation, which is to cauterize a biological tissue by a high-frequency current from the electrode portion. As a treatment by ablation, the shunt treatment on the atrial septum is known. For the patients who suffer from heart failure, by forming, in an atrial septum, a shunt (puncture hole) serving as an escape route for increased atrial pressure, the shunt treatment enables heart failure symptoms to be alleviated. In the shunt treatment, the atrial septum is accessed using an intravenous approaching method, and the puncture hole is formed to have a desired size.

In a medical device in which a plurality of electrode portions are arranged on an expansion body, when cauterization is started in a state in which the electrode portions and a biological tissue are not sufficiently in contact with each other, sufficient tissue cauterization cannot be obtained, and treatment may become insufficient. In this case, the electrode portions are exposed to blood, which causes thrombus formation.

Patent Literature 1 discloses a medical device for performing ablation, in order to detect a contact state of an electrode portion with respect to a biological tissue, the medical device measuring impedance of each electrode portion in a state in which the electrode portion is arranged at a predetermined position in a biological body.

### Citation List

### Patent Literature

Patent Literature 1: WO 2019-85841 A

### Summary of Invention

### Technical Problem

As in Patent Literature 1, when the impedance of each electrode portion is measured, a difference between the impedance of the electrode portion in contact with a biological tissue and the impedance of the electrode portion not in contact with the biological tissue is not detected much significant, and thus it is sometimes difficult to detect the contact state of the electrode portions. In particular, in a monopolar electrode to which a voltage is applied between the monopolar electrode and a body surface electrode, the difference in impedance due to the contact state of the electrode portion is much smaller than the original impedance, and thus, it is more difficult to detect the contact state.

The present invention has been made to solve the above-described problems, and an object is to provide a medical device system and an electrode portion contact state detection method configured to reliably detect a contact state of each electrode portion with respect to a biological tissue in a case where the medical device system includes three or more electrode portions.

### Solution to Problem

A medical device system according to the present invention for achieving the above object includes: a medical device including three or more electrode portions to be inserted into a biological body; and a measurement unit that measures impedance among the electrode portions, in which the measurement unit, by selecting all the electrode portions one by one, performs a measurement operation of measuring impedance between one end of a selected electrode portion from any one of the electrode portions and one end of a parallel circuit of all the electrode portions other than the selected electrode portion.

A shunt forming method according to the present invention for achieving the above object is an electrode portion contact detection method for detecting a contact state of three or more electrode portions included in a medical device with respect to a biological tissue, the electrode portion contact detection method including: providing a measurement unit that measures impedance among the electrode portions, and causing the measurement unit to select any one of the electrode portions and to perform a measurement operation of measuring impedance between one end of the selected electrode portion and one end of a parallel circuit of all the electrode portions other than the selected electrode portion by selecting all the electrode portions one by one.

### Advantageous Effects of Invention

In the medical device system and the electrode portion contact detection method configured as described above, the impedance measured by the measurement unit greatly reflects the impedance of the electrode portion having been selected, and thus it is possible to reliably detect whether or not the electrode portion having been selected is in contact with a biological tissue.

The electrode portion may be disposed on an expansion body configured to expand and contract in a radial direction. This makes it possible to detect the contact state of the electrode portion arranged on the expansion body.

The expansion body may include a recessed portion recessed radially inward and defining a receiving space configured to receive a biological tissue when the expansion body expands, the recessed portion may include a bottom portion positioned on a radial innermost side, a proximal side upright portion extending radially outward from a proximal end of the bottom portion, and a distal side upright portion extending radially outward from a distal end of the bottom portion, and the electrode portions may be arranged in the proximal side upright portion or the distal side upright portion in such a manner that the electrode portions each face the receiving space. This makes it possible to detect a contact state of the electrode portions with respect to the biological tissue received in the receiving space.

The frequency of the voltage applied to the electrode portions when the measurement unit performs the measurement operation may be in a range of 300 kHz to 13.56 MHz. This frequency range has a large difference in resistivity between the blood and the biological tissue, and thus a large difference occurs in the impedance to be measured depending on whether or not the electrode portions are in contact with the biological tissue, and the contact state of the electrode portions can be determined more clearly.

### Brief Description of Drawings

Fig. 1 is a front view illustrating an overall configuration of a medical device according to an embodiment.
Fig. 2 is an enlarged perspective view illustrating the vicinity of an expansion body.
Fig. 3 is an enlarged front view of the vicinity of the expansion body.
Fig. 4 is a view illustrating connection of a circuit for measuring impedance with a measurement unit.
Fig. 5 is a view for schematically describing a state in which the expansion body is disposed in the atrial septum, in which the medical device is illustrated in a front view and the biological tissue is illustrated in a sectional view, respectively.
Fig. 6 is an enlarged view of the vicinity of the expansion body in Fig. 5.
Fig. 7 is a view for describing a state in which the diameter of the expansion body is increased in the atrial septum from the state of Fig. 6.
Fig. 8 is a flowchart of a treatment method using a medical device.
Fig. 9 is an enlarged view of the vicinity of an expansion body of a first modification example.
Fig. 10 is an enlarged view of the vicinity of an expansion body of a second modification example.
Fig. 11 is an enlarged view of the vicinity of an expansion body of a third modification example.
Fig. 12 is an enlarged view of the vicinity of an expansion body of a fourth modification example.
Fig. 13 is an enlarged view of the vicinity of an expansion body of a fifth modification example.

### Description of Embodiment

Hereinafter, an embodiment of the present invention will be described with reference to the drawings. In some cases, dimensional ratios in the drawings may be exaggerated and different from actual ratios for convenience of description. In addition, in the present specification, a side on which a medical device 10 is inserted into a biological lumen will be referred to as a "distal end" or a "distal side", and an operating hand-side will be referred to as a "proximal end" or a "proximal side".

A medical device system according to the embodiment below includes a medical device 10 and a measurement unit 60. The medical device 10 is configured as follows. A puncture hole Hh formed in an atrial septum HA of a patient's heart H is enlarged, and further, a maintenance treatment is performed so that the puncture hole Hh having an increased diameter is maintained to have an increased size. The measurement unit 60 detects a contact state of an electrode portion 22 included in the medical device 10 with respect to a biological tissue.

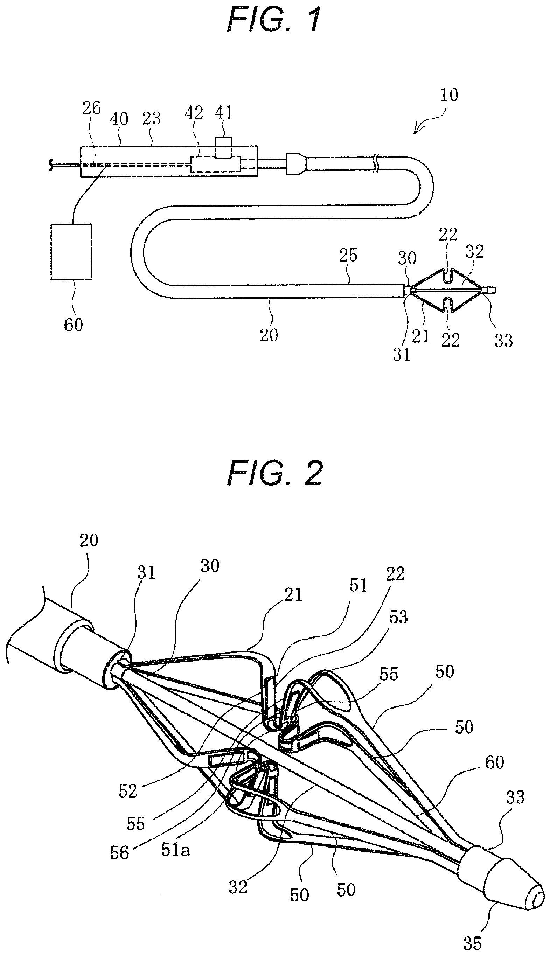

As illustrated in Fig. 1, the medical device 10 included in the medical device system of the present embodiment includes an elongated shaft portion 20, an expansion body 21 disposed in a distal portion of the shaft portion 20, and an operation unit 23 disposed in a proximal portion of the shaft portion 20. The expansion body 21 has an electrode portion 22, which is an energy transfer element for performing the above-described maintenance treatment.

The shaft portion 20 has a distal portion 30 including a proximal end fixing portion 31 to which a proximal end of the expansion body 21 is fixed and a distal end fixing portion 33 to which a distal end of the expansion body 21 is fixed. The distal portion 30 of the shaft portion 20 has a shaft extension portion 32 extending in the expansion body 21 from the proximal end fixing portion 31. The shaft portion 20 has a storage sheath 25 disposed at an outermost peripheral portion. The expansion body 21 is movable forward and rearward from the storage sheath 25 in an axial direction. In a state where the storage sheath 25 is moved to the distal side of the shaft portion 20, the storage sheath 25 can internally store the expansion body 21. In a state where the expansion body 21 is stored, the storage sheath 25 is moved to the proximal side. In this manner, the expansion body 21 can be exposed.

The shaft portion 20 includes a pulling shaft 26. The pulling shaft 26 is disposed from the proximal end of the shaft portion 20 to the shaft extension portion 32, and the distal portion is fixed to a distal member 35. A guide wire lumen is formed in the pulling shaft 26 and the distal member 35 along the axial direction, and a guide wire 11 can be inserted into the guide wire lumen.

The distal member 35 to which the distal portion of the pulling shaft 26 is fixed needs not be fixed to the expansion body 21. In this manner, the distal member 35 can pull the expansion body 21 in a contracting direction. In addition, when the expansion body 21 is stored in the storage sheath 25, the distal member 35 is separated to the distal side from the expansion body 21. Accordingly, the expansion body 21 can be rather easily moved in an axial direction, and storage capability can be improved.

The operation unit 23 has a housing 40 configured to be held by an operator, an operation dial 41 that can be rotationally operated by the operator, and a conversion mechanism 42 operated in conjunction with the rotation of the operation dial 41. The pulling shaft 26 is held inside the operation unit 23 by the conversion mechanism 42. In conjunction with the rotation of the operation dial 41, the conversion mechanism 42 can move the held pulling shaft 26 forward and backward along the axial direction. For example, a rack and pinion mechanism can be used as the conversion mechanism 42.

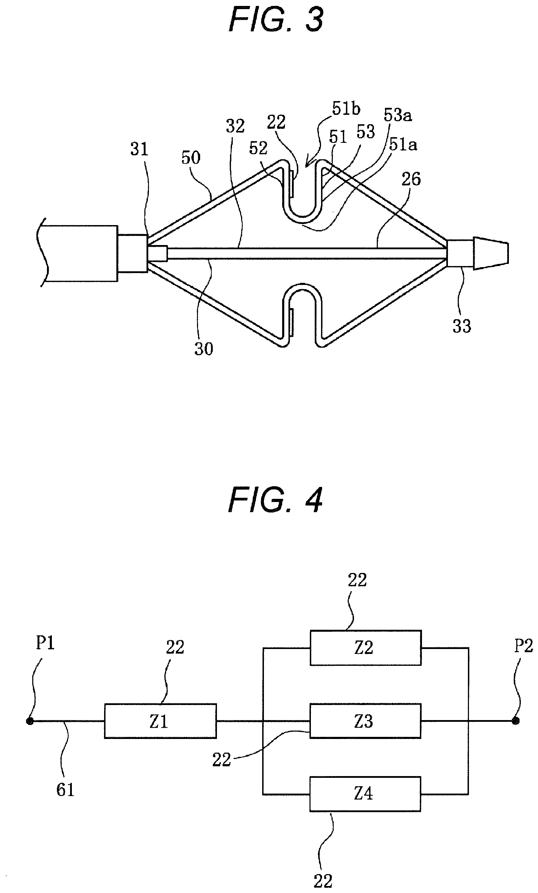

The expansion body 21 will be described in more detail. As shown in Figs. 2 and 3, the expansion body 21 has a plurality of wire portions 50 in a circumferential direction. In the present embodiment, for example, four of the wire portions 50 are disposed in the circumferential direction. The wire portions 50 are respectively configured to expand and contract in a radial direction. A proximal portion of the wire portion 50 extends to a distal side from the proximal end fixing portion 31. A distal portion of the wire portion 50 extends from a proximal portion of the distal member 35 to a proximal side. The wire portion 50 is inclined to increase in the radial direction from both end portions toward a central portion in an axial direction. In addition, in the wire portion 50, the central portion in the axial direction has a recessed portion 51 recessed radially inward of the expansion body 21. A radially innermost portion of the recessed portion 51 is a bottom portion 51a. The recessed portion 51 defines a receiving space 51b configured to receive a biological tissue when the expansion body 21 expands.

The recessed portion 51 includes a proximal side upright portion 52 extending radially outward from the proximal end of the bottom portion 51a and a distal side upright portion 53 extending radially outward from the distal end of the bottom portion 51a. In the distal side upright portion 53, a central portion in a width direction has a slit shape. The distal side upright portion 53 has an outer edge portion 55 on both sides and a backrest portion 56 of the central portion.

For example, the wire portion 50 forming the expansion body 21 has a flat plate shape cut out from a cylinder. The wire forming the expansion body 21 can have, for example, a thickness of a range of 50 um to 500 um and a width of a range of 0.3 mm to 2.0 mm. However, the wire may have a dimension outside this range. In addition, the wire portion 50 may have a circular shape in a cross section, or may have other shapes in a cross section.

The electrode portion 22 is disposed along the proximal side upright portion 52. Accordingly, when the recessed portion 51 is disposed in the atrial septum HA, the energy from the electrode portion 22 may be transferred to the atrial septum HA from the right atrium side.

For example, the electrode portion 22 is configured to include a bipolar electrode that receives electric energy from an energy supply device (not illustrated) serving as an external device. In this case, electricity is supplied to the electrode portion 22 disposed in each of the wire portions 50. The electrode portion 22 and the energy supply device are connected to each other by a conducting wire (not illustrated) coated with an insulating coating material. The conducting wire is drawn outward (i.e., extends) via the shaft portion 20 and the operation unit 23, and is connected to the energy supply device.

Alternatively, the electrode portion 22 may be configured to serve as a monopolar electrode. In this case, the electricity is supplied from a counter electrode plate prepared outside a body. Even when configured to serve as a monopolar electrode, the electrode portion 22 serves as a bipolar electrode configured to supply electricity between the electrode portions 22 when the detection unit 60 measures the impedance. In addition, the electrode portion 22 may alternatively be a heating element (electrode chip) that generates heat by receiving high-frequency electric energy from the energy supply device. In this case, the electricity is supplied to the heating element disposed in each of the wire portions 50. Furthermore, the electrode portion 22 can be configured to include an energy transfer element that applies energy to the puncture hole Hh, such as a heater including an electric wire which provides heating and cooling operation or generating frictional heat by using microwave energy, ultrasound energy, coherent light such as laser, a heated fluid, a cooled fluid, or a chemical medium. A specific form of the energy transfer element is not particularly limited. In this manner, in a case where energy is applied to a biological tissue with a configuration other than the electrode portion 22, an electrode portion for contact detection is disposed in the vicinity of a place where the energy is applied, and is connected to the detection unit 60.

The wire portion 50 is configured to be formed of a metal material. For example, the metal material can be a titanium-based (Ti-Ni, Ti-Pd, or Ti-Nb-Sn) alloy, a copper-based alloy, stainless steel, β-titanium steel, or a Co-Cr alloy. An alloy having a spring property such as a nickel titanium alloy may also be used as the material of the wire portion. However, a material of the wire portion 50 is not limited, and the wire portion 50 may be formed of other materials.

It is preferable that the shaft portion 20 is formed of a material having a certain degree of flexibility. For example, the materials of the shaft portion 20 may include polyolefin such as polyethylene, polypropylene, polybutene, ethylene-propylene copolymer, ethylene-vinyl acetate copolymer, ionomer, and a mixture of the above-described two or more materials, fluororesin such as soft polyvinyl chloride resin, polyamide, polyamide elastomer, polyester, polyester elastomer, polyurethane, and polytetrafluoroethylene, polyimide, PEEK, silicone rubber, or latex rubber.

For example, the pulling shaft 26 can be formed of the materials in which an elongated wire formed of a super elastic alloy such as a nickel-titanium alloy and a copper-zinc alloy, a metal material such as stainless steel, or a resin material having relatively high rigidity is coated with a resin material such as polyvinyl chloride, polyethylene, polypropylene, and ethylene-propylene copolymer.

For example, the distal member 35 can be formed of a polymer material such as polyolefin, polyvinyl chloride, polyamide, polyamide elastomer, polyurethane, polyurethane elastomer, polyimide, and fluororesin or a mixture of polymer materials. Alternatively, the distal member 35 can be formed of a multilayer tube containing two or more polymer materials.

The measurement unit 60 will be described. The measurement unit 60 is connected to each of the electrode portions 22 by a line 61, and is configured to measure the impedance of the connected electrode portion 22. The measurement unit 60 is configured to select one of four electrode portions 22 and connect the other three electrode portions 22 in parallel. The measurement unit 60 performs a measurement operation of measuring the impedance between one end P1 of the electrode portion 22 having been selected and one end P2 of the parallel circuit in which the other three electrode portions 22 are connected in parallel. In this manner, as illustrated in Fig. 4, the measurement unit 60 measures an impedance in which an impedance Z1 of the electrode portion 22 having been selected and an impedance in which impedances Z2, Z3, and Z4 of the other three electrode portions 22 are synthesized in parallel are synthesized in series. The measurement operation is performed four times in total by selecting the four electrode portions 22 one by one.

Since the measurement unit 60 measures the series synthetic impedance of one of the electrode portions 22 and the parallel circuit in which the remaining electrode portions 22 are connected in parallel, the impedance Z to be measured is expressed by Z1 + (1/Z2 + 1/Z3 + 1/Z4)⁻¹. That is, the impedance Z to be measured is greatly affected by the impedance of the electrode portion 22 having been selected. This makes it possible to clearly determine whether the impedance of the electrode portion 22 having been selected is in a state in which the impedance is high due to contact with the biological tissue or in a state in which the impedance is low due to non-contact with the biological tissue and contact with the blood.

The measurement unit 60 supplies a high-frequency current to each of the electrode portions 22 in order to measure the impedance of the electrode portions 22. As the frequency of this high-frequency current, a region with a range of 300 kHz to 13.56 MHz can be used. At a frequency in this range, the difference between the resistivity of the blood and the resistivity of the biological tissue is so large that the difference between a high impedance indicated by the electrode portions 22 in contact with the biological tissue and a low impedance indicated by the electrode portions 22 not in contact with the biological tissue but in contact with the blood becomes large. Therefore, the contact state of the electrode portions 22 can be determined more clearly.

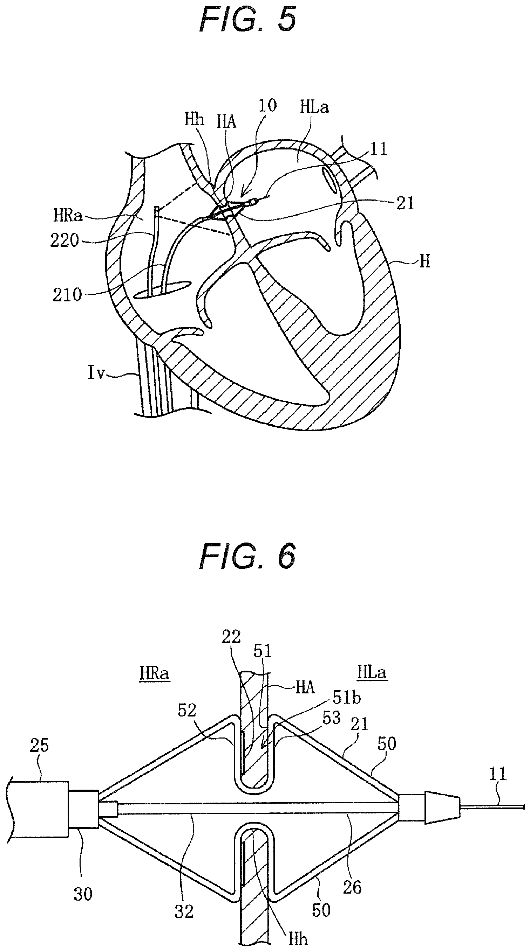

The treatment method using the medical device 10 will be described. The treatment method according to the present embodiment is performed on a patient suffering from a heart failure (left heart failure). More specifically, as shown in Fig. 5, the treatment method is performed on the patient suffering from a chronic heart failure, who has high blood pressure in a left atrium HLa due to myocardial hypertrophy appearing in a left ventricle of the heart H and increased stiffness (hardness).

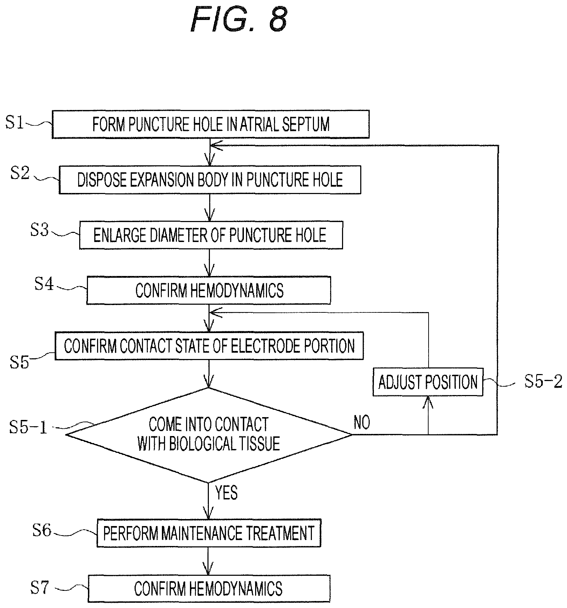

As presented in Fig. 8, the treatment method according to the present embodiment includes forming the puncture hole Hh in the atrial septum HA (S1), disposing the expansion body 21 in the puncture hole Hh (S2), enlarging the diameter of the puncture hole Hh by using the expansion body 21 (S3), confirming hemodynamics in the vicinity of the puncture hole Hh (S4), confirming the contact state of the electrode portions 22 with respect to the biological tissue (S5), performing the maintenance treatment for maintaining the size of the puncture hole Hh (S6), and confirming the hemodynamics in the vicinity of the puncture hole Hh after the maintenance treatment is performed (S7).

When the puncture hole Hh is formed, an operator delivers an introducer 210 in which a guiding sheath and a dilator are combined with each other, to the vicinity of the atrial septum HA. For example, the introducer 210 can be delivered to a right atrium HRa via an inferior vena cava Iv. In addition, the introducer can be delivered using the guide wire 11. The operator can insert the guide wire 11 into the dilator, and can deliver the introducer along the guide wire 11. The introducer and the guide wire 11 can be inserted into a living body by using a known method such as using a blood vessel introducer.

In the forming of the puncture hole Hh in the atrial septum HA (S1), the operator causes a puncture device (not illustrated) to penetrate from the right atrium HRa side toward the left atrium HLa side, thereby forming the puncture hole Hh. For example, a device such as a wire having a sharp distal end can be used as the puncture device. The puncture device is inserted into the dilator, and is delivered to the atrial septum HA. The puncture device can be delivered to the atrial septum HA instead of the guide wire 11 after the guide wire 11 is removed from the dilator.

In the enlarging of the diameter of the puncture hole Hh by using the expansion body 21 (S2), the medical device 10 is first delivered to the vicinity of the atrial septum HA along the guide wire 11 inserted in advance. At this time, the distal portion of the medical device 10 penetrates the atrial septum HA, and reaches the left atrium HLa. In addition, when the medical device 10 is inserted, the expansion body 21 is in a state of being stored in the storage sheath 25.

Next, as shown in Fig. 6, the storage sheath 25 is moved to the proximal side so that the expansion body 21 is exposed. In this manner, the diameter of the expansion body 21 increases, and the recessed portion 51 is arranged in the puncture hole Hh of the atrial septum HA and receives the biological tissue surrounding the puncture hole Hh in the receiving space 51b. In this manner, the biological tissue is held between the electrode portion 22 and the distal side upright portion 53 having an opposing surface portion 53a.

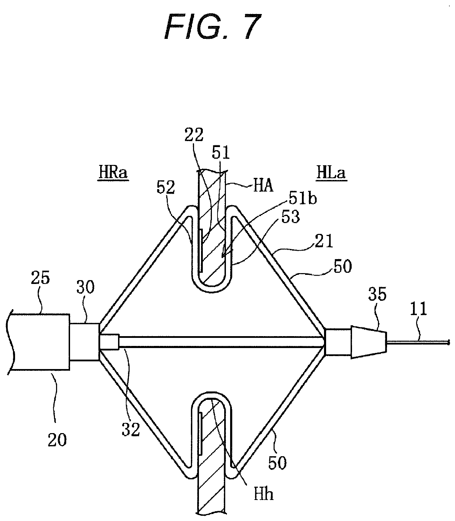

In the enlarging of the diameter of the puncture hole Hh by using the expansion body 21 (S3), the operator operates the operation unit 23 in a state where the receiving space 51b receives the biological tissue, and the pulling shaft 26 is moved to the proximal side. In this manner, as shown in Fig. 7, the expansion body 21 further expands in the radial direction, and the puncture hole Hh is widened in the radial direction.

After the puncture hole Hh is enlarged, the hemodynamics is confirmed in the vicinity of the puncture hole Hh (S4). As shown in Fig. 5, the operator delivers a hemodynamics confirming device 220 to the right atrium HRa by way of the inferior vena cava Iv. For example, a known echo catheter can be used as the hemodynamics confirming device 220. The operator can display an echo image acquired by the hemodynamics confirming device 220 on a display apparatus such as a display, and can confirm a blood volume passing through the puncture hole Hh, based on a result of the echo image.

Before cauterization by the electrode portion 22, in confirming the contact state of the electrode portions 22 with respect to the biological tissue (S5), the measurement unit 60 determines whether each of the electrode portions 22 is in contact with the biological tissue. The measurement unit 60 selects the electrode portions 22 one by one, and performs the measurement operation described above. When the measured impedance is smaller than a certain reference value, it is determined that the electrode portion 22 having been selected is not in contact with the biological tissue. The reference value of the impedance can be set in advance based on a known impedance of the blood and impedance of the biological tissue. The impedance of the blood may be acquired by measuring the impedance between the electrode portions 22 in a state in which the electrode portions 22 are exposed to the blood before the electrode portions 22 are brought into contact with the biological tissue, for example, between steps S1 and S2, and the reference value of the impedance may be set on the basis of this value. For the setting of the reference value, the latter can absorb variation in the impedance of the blood for each patient, and thus the contact state can be detected more accurately.

If some of the electrode portions 22 are not in contact with the biological tissue, the operator adjusts the position so that the expansion body 21 comes into contact with the biological tissue by performing again from the step of S2 or moving, rotating, or the like the shaft portion 20 in an axial direction (S5-2). Then, the measurement unit 60 performs the measurement operation again to confirm the contact state of the electrode portions 22.

When it is confirmed in step S5 that all the electrode portions 22 are in contact with the biological tissue, the operator performs the maintenance treatment for maintaining the size of the puncture hole Hh (S6). In the maintenance treatment, high-frequency energy is applied to an edge portion of the puncture hole Hh through the electrode portion 22, thereby cauterizing (heating and cauterizing) the edge portion of the puncture hole Hh by using the high-frequency energy.

When the biological tissue in the vicinity of the edge portion of the puncture hole Hh is cauterized through the electrode portion 22, a degenerated portion having the degenerated biological tissue is formed in the vicinity of the edge portion. The biological tissue in the degenerated portion is in a state where elasticity is lost. Accordingly, the puncture hole Hh can maintain a shape widened by the expansion body 21.

After the maintenance treatment is performed, the hemodynamics are confirmed again in the vicinity of the puncture hole Hh after the maintenance treatment (S7). In a case where the blood volume passing through the puncture hole Hh reaches a desired volume, the operator decreases the diameter of the expansion body 21. After the expansion body 21 is stored in the storage sheath 25, the expansion body 21 is removed from the puncture hole Hh. Furthermore, the whole medical device 10 is removed outward of the living body, and the treatment is completed.

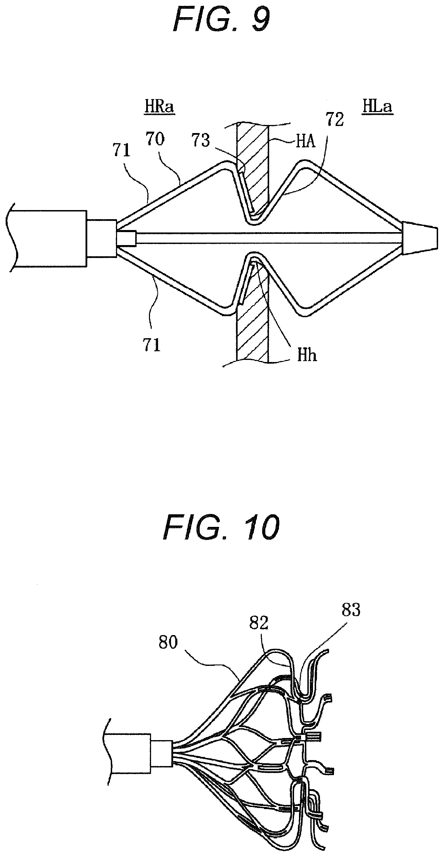

Next, modification examples of the expansion body will be described. As illustrated in Fig. 9, an expansion body 70 of the first modification example is formed of a plurality of wire portions 71, and each of the wire portions 71 has a recessed portion 72 that receives a biological tissue. An electrode portion 73 is disposed in the recessed portion 72. The expansion body 70 expands in the radial direction, but does not grip a biological tissue, and brings the electrode portion 73 into contact with the biological tissue by pressing the recessed portion 72 against the puncture hole Hh. As described above, also in the expansion body 70 having the recessed portion 72 but not gripping, the measurement unit 60 is configured to measure the impedance of the electrode portion 73, and it is possible to detect the contact state of the electrode portion 73 with respect to the biological tissue.

As shown in Fig. 10, an expansion body 80 of the second modification example is formed in a mesh shape in which wires are branched and merged. The expansion body 80 has a recessed portion 82 that receives a biological tissue. An electrode portion 83 is disposed in the recessed portion 82. This expansion body 80 does not have a part distal of the recessed portion 82. In the present example, the shaft portion does not have a pulling shaft, and the puncture hole Hh can be expanded only by the self-expansion force of the expansion body 80. Also in such an expansion body 80, the measurement unit 60 is configured to measure the impedance of the electrode portion 83, and it is possible to detect the contact state of the electrode portion 83 with respect to the biological tissue.

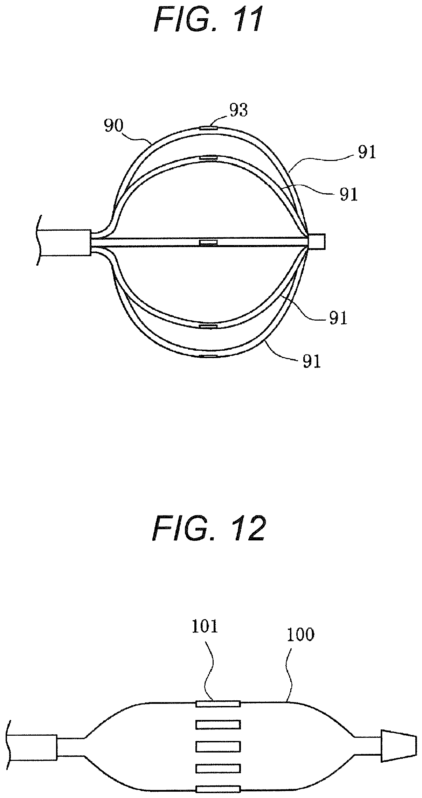

As illustrated in Fig. 11, an expansion body 90 of the third modification example includes a plurality of wire portions 91. The wire portion 91 does not have a recessed portion, and has an electrode portion 93 at a part positioned on the radial outermost side. As described above, even in the expansion body 90 having no recessed portion, the measurement unit 60 is configured to measure the impedance of the electrode portion 93, and it is possible to detect the contact state of the electrode portion 93 with respect to the biological tissue.

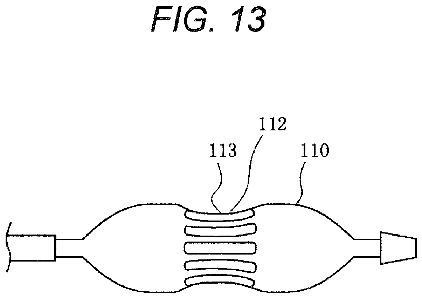

As illustrated in Fig. 12, an expansion body 100 of the fourth modification example is a balloon, and a plurality of electrode portions 103 are arranged on the outer surface of the balloon along the circumferential direction. The expansion body 100 does not have a recessed portion. As described above, also in the expansion body 100 formed of a balloon having no recessed portion, the measurement unit 60 is configured to measure the impedance of the electrode portion 103, and it is possible to detect the contact state of the electrode portion 103 with respect to the biological tissue. As illustrated in Fig. 13, an expansion body 110 formed of a balloon and having an electrode portion 113 on the outer surface may have a recessed portion 112.

As described above, the medical device system according to the present embodiment includes: the medical device 10 including three or more of the electrode portions 22 to be inserted into a biological body; and the measurement unit 60 that measures impedance among the electrode portions 22, in which the measurement unit 60, by selecting all the electrode portions 22 one by one, performs a measurement operation of measuring impedance between one end of a selected electrode portion 22 from any one of the electrode portions 22 and one end of a parallel circuit of all the electrode portions 22 other than the selected electrode portion 22. In the medical device system configured as described above, since the impedance measured by the measurement unit 60 greatly reflects the impedance of the electrode portion 22 having been selected, it is possible to reliably detect whether or not the electrode portion 22 having been selected is in contact with a biological tissue.

The electrode portions 22 may be disposed on the expansion body 21 configured to expand and contract in a radial direction. This makes it possible to detect the contact state of the electrode portion 22 arranged on the expansion body 21.

The expansion body 21 may include the recessed portion 51 recessed radially inward and defining the receiving space 51b configured to receive a biological tissue when the expansion body 21 expands, the recessed portion 51 may include the bottom portion 51a positioned on a radial innermost side, the proximal side upright portion 52 extending radially outward from a proximal end of the bottom portion 51a, and the distal side upright portion 53 extending radially outward from a distal end of the bottom portion 51a, and the electrode portions 22 may be arranged in the proximal side upright portion 52 or the distal side upright portion 53 in such a manner that the electrode portions 22 each face the receiving space 51b. This makes it possible to detect a contact state of the electrode portions 22 with respect to the biological tissue received in the receiving space 51b.

The frequency of the voltage applied to the electrode portions 22 when the measurement unit 60 performs the measurement operation may be in a range of 300 kHz to 13.56 MHz. This frequency range has a large difference in resistivity between the blood and the biological tissue, and thus a large difference occurs in the impedance to be measured depending on whether or not the electrode portions 22 are in contact with the biological tissue, and the contact state of the electrode portions 22 can be determined more clearly.

The shunt forming method according to the present embodiment is an electrode portion contact detection method for detecting a contact state of three or more of the electrode portions 22 included in the medical device 10 with respect to a biological tissue, disposing the measurement unit 60 for measuring impedance between the electrode portions 22, and, by selecting all the electrode portions 22 one by one, performing a measurement operation of measuring impedance between one end of a selected electrode portion 22 from any one of the electrode portions 22 and one end of a parallel circuit of all the electrode portions 22 other than the selected electrode portion 22. In the electrode portion contact detection method configured as described above, since the impedance measured by the measurement unit 60 greatly reflects the impedance of the electrode portion 22 having been selected, it is possible to reliably detect whether or not the electrode portion 22 having been selected is in contact with a biological tissue.

The present invention is not limited to the above-described embodiments, and various modifications can be made by those skilled in the art within the technical idea of the present invention. In the above-described embodiment, four of the electrode portions 22 are disposed in the circumferential direction, but the number of electrode portions 22 may be any number as long as it is three or more.

This application is based on Japanese Application No. 2020-164556 filed on September 30, 2020, the entire content of which is incorporated herein by reference.

### Reference Signs List

- 10: medical device
- 11: guide wire
- 20: shaft portion
- 21: expansion body
- 22: electrode portion
- 23: operation unit
- 25: storage sheath
- 26: pulling shaft
- 30: distal portion
- 31: proximal end fixing portion
- 32: shaft extension portion
- 33: distal end fixing portion
- 35: distal member
- 40: housing
- 41: operation dial
- 42: conversion mechanism
- 50: wire portion
- 51: recessed portion
- 51a: receiving space
- 52: bottom portion
- 53: proximal side upright portion
- 54: distal side upright portion
- 55: outer edge portion
- 56: backrest portion
- 57: through-hole
- 58: space portion
- 60: measurement unit
- 61: line

## Claims

1. A medical device system comprising:
a medical device including three or more electrode portions to be inserted into a biological body; and
a measurement unit configured to measure impedance among the electrode portions, wherein
the measurement unit is configured to, by selecting all the electrode portions one by one, perform a measurement operation of measuring impedance between one end of a selected electrode portion from any one of the electrode portions and one end of a parallel circuit of all the electrode portions other than the selected electrode portion.

2. The medical device system according to claim 1, wherein the electrode portions are arranged at an expansion body configured to expand and contract in a radial direction.

3. The medical device system according to claim 2, wherein
the expansion body includes a recessed portion recessed radially inward and defining a receiving space configured to receive a biological tissue when the expansion body expands,
the recessed portion includes a bottom portion positioned on a radial innermost side, a proximal side upright portion extending radially outward from a proximal end of the bottom portion, and a distal side upright portion extending radially outward from a distal end of the bottom portion, and
the electrode portions are arranged in the proximal side upright portion or the distal side upright portion in such a manner that the electrode portions each face the receiving space.

4. The medical device system according to any one of claims 1 to 3, wherein a frequency of a voltage applied to the electrode portions when the measurement unit performs the measurement operation is in a range of 300 kHz to 13.56 MHz.

5. An electrode portion contact detection method for detecting a contact state of three or more electrode portions included in a medical device with respect to a biological tissue, the electrode portion contact detection method comprising:
providing a measurement unit that measures impedance among the electrode portions; and
causing the measurement unit to select any one of the electrode portions and to perform a measurement operation of measuring impedance between one end of the selected electrode portion and one end of a parallel circuit of all the electrode portions other than the selected electrode portion by selecting all the electrode portions one by one.
